# DEMANDE DE BREVET EUROPEEN

(11) **EP 0 811 664 A1**
(43) Date de publication de la demande: **10.12.1997**
(21) Numéro de dépôt: 97401141.3
(22) Date de dépôt: 26.05.1997
(51) Int. Cl.: C09D 193/04, C08L 93/04, A23P 1/08, A23C 19/16, A61K 9/28

(54) **Composition d'enrobage et utilisations dans l'industrie alimentaire et dans l'industrie pharmaceutique**

(30) Priorité: 07.06.1996 FR 9607254
(71) Demandeur: Société Industrielle des Oleagineux, 62053 Saint Laurent-Blangy (FR)
(72) Inventeur: Dauthy, Mircea, 77186 Noisiel (FR)

(57) **Abrégé**

Des compositions comprenant 20-80 % de mono-diglycérides d'acides gras, de 3 à 30 % en poids d'ester méthylique de colophane hydrogénée ou de colophane hydrogénée et esterifiée au glycérol et optionnellement des monoglycérides acétylés à 50 % et des monoglycérides acétylés à 70 %, du polyisobutylène (50 000-80 000) et d'autres matières grasses hydrogénées d'origine végétale ou animale, sont utilisables pour enrober par application directe un produit alimentaire ou pour former par imprégnation de feuilles de papier ou de textiles des feuilles d'emballage.

## Description

La présente invention concerne des compositions d'enrobage, leurs procédés de préparation et des utilisations de ces compositions.

Pour protéger des agressions extérieures les fromages sans croûtes, voire même des fromages à croûtes, il est connu de les revêtir d'une couche plus ou moins épaisse de paraffine colorée ou non. Un tel mode de protection des fromages est décrit par exemple dans le brevet FR 1 453 977.

Il est également connu d'incorporer dans ces couches protectrices un ruban qui en dépasse légèrement et qui, lorsque l'on tire dessus, provoque la déchirure de la couche protectrice. La mise en place de tels rubans est décrite par exemple dans le brevet FR 2 604 340.

Les pellicules de protection mentionnées ci-dessus sont normalement réalisées à base de paraffine. Cependant au cours de ces dernières années, les législations de plusieurs pays tendent à limiter l'utilisation des huiles de paraffine et d'autres dérivés d'hydrocarbures minéraux dans les produits alimentaires, pharmaceutiques et cosmétiques, pour des raisons de santé.

Les brevets EP 403 030 et EP 679 337 décrivent des procédés d'enrobage de fromage utilisant des compositions d'enrobage à base de mélanges de monoglycérides, de diglycérides ou de monoglycérides acétylés d'acides gras, d'origine animale ou végétale.

Pour l'enrobage de fromages, on utilise fréquemment une technique de trempage en deux étapes, dans laquelle on fabrique une première couche par trempage du fromage dans la composition d'enrobage chauffée et liquéfiée, puis égouttage et refroidissement, puis ensuite une deuxième couche, également par trempage, égouttage et refroidissement. Pour la formation de la deuxième couche, on trempe le fromage déjà revêtu de sa première couche solidifiée dans un deuxième bain de matière d'enrobage fondue, qui peut être identique ou différente du matériau de la première couche.

Les compositions connues dans l'état de la technique présentent quelques inconvénients pour la mise en oeuvre de ce procédé ; l'épaisseur de la première couche obtenue n'est souvent pas uniforme. En particulier, lors de l'égouttage, il se forme fréquemment ce qu'on appelle "une goutte", c'est-à-dire une surépaisseur locale trop importante au point le plus bas de la pièce de fromage. Le défaut d'uniformité de l'enrobage peut alors se trouver amplifié lors de la formation de la deuxième couche, si le fromage reste dans la même disposition dans le processus de trempage suivant.

De plus, certaines compositions de l'art antérieur présentent des défauts à l'ouverture, c'est-à-dire que lorsqu'on tire sur le ruban, la couche d'enrobage se déchire suivant une ligne brisée irrégulière et non suivant une ligne régulière contigue au ruban.

Enfin le point de fusion de certaines compositions connues est assez élevé, ce qui impose une consommation d'énergie non négligeable, ces compositions étant mises en oeuvre à l'état liquide, ainsi qu'un risque de détérioration des propriétés organoleptiques des produits alimentaires enrobés.

Le but de la présente invention est de remédier à ces inconvénients de l'art antérieur.

Ce but est atteint conformément à l'invention grâce à une composition d'enrobage qui comprend de 20 à 80 % en poids de mono-diglycérides d'acides gras et de 3 % à 30 % en poids d'une substance résineuse choisie parmi les esters méthyliques de colophane hydrogénée et désodorisée, la colophane hydrogénée estérifiée au glycérol et désodorisée, et leurs mélanges.

Les mono-diglycérides d'acides gras peuvent être choisis parmi les produits fabriqués à partir de matières grasses végétales ou animales. Ils peuvent aussi être fabriqués à partir d'acides gras eux-mêmes. De préférence, les acides gras sont l'acide myristique (C14:0), le palmitique (C16:0), le stéarique (C18:0), l'arachique (C20:0) et le béhénique (C22:0).

De préférence, les compositions d'enrobage selon l'invention comprennent entre 20 % à 50 % en poids desdits mono-diglycérides, et de 25 % à 70 % en poids d'un ou plusieurs glycérides acétylés choisis parmi les monoglycérides acétylés à 50 % et les monoglycérides acétylés à 70 %.

De manière particulièrement préférée, les compositions contiennent une association de monoglycérides acétylés à 50 % et de monoglycérides acétylés à 70 % et le rapport pondéral monoglycérides acétylés à 50 %/monoglycérides acétylés à 70 % est compris entre 0,6 et 5,7 et de préférence entre 1,4 et 3,8.

En plus des substances de base ci-dessus, les compositions selon l'invention peuvent contenir une ou plusieurs substances choisies parmi la lécithine de soja, l'oléine de palme hydrogénée, l'huile de maïs hydrogénée, l'huile de palmiste hydrogénée, les matières grasses végétales hydrogénées et interesterifiées.

Parmi les composants de base, les compositions d'enrobage selon l'invention peuvent également contenir de 10 à 36 % en poids de polyisobutylène d'un poids moléculaire compris entre 10000 et 95000 et de préférence entre 50000 et 80000.

Les compositions d'enrobage selon l'invention sont utilisées de façon particulièrement avantageuse pour l'enrobage d'un produit alimentaire, notamment d'un fromage ou d'une préparation fromagère.

Cet enrobage peut être constitué d'une couche ou de deux couches de la même composition ou encore de deux couches de composition différente.

Les compositions d'enrobage selon l'invention peuvent également être utilisées avantageusement pour la fabrication d'un produit d'emballage en feuilles. Dans cette utilisation, on imprègne un support choisi de préférence parmi les papiers, les cartons, les textiles tissés ou non-tissés d'une composition d'enrobage selon l'invention.

Les compositions d'enrobage selon l'invention peuvent également avantageusement être utilisées pour la protection, à savoir enrobage ou emballage, d'un produit cosmétique ou d'un produit pharmaceutique.

L'invention utilise donc des substances résineuses d'origine naturelle, à savoir des esters méthyliques de colophane hydrogénée et désodorisée et/ou de la colophane hydrogénée estérifiée à la glycérine et désodorisée, associés à des mélanges divers de dérivés de matières grasses végétales et, dans certaines compositions, au polyisobutylène et à la lécithine de soja.

De manière surprenante, l'utilisation des esters méthyliques de colophane hydrogénée et désodorisée et/ou de la colophane hydrogénée estérifiée à la glycérine et désodorisée dans les compositions selon la présente invention permet à celles-ci d'être utilisées pour la protection et l'emballage de produits alimentaires, ces revêtements de protection et emballages ayant une consistance solide ou semi-solide, agréable au toucher et agréable à l'oeil.

La présente invention sera mieux comprise de l'homme du métier grâce à la description détaillée des composants utilisés, de procédés utilisés pour la préparation des compositions selon l'invention ainsi que des exemples d'utilisation. Les dénominations en lettres majuscules identifiant des composés chimiques désignent des marques ou des noms commerciaux.

Les esters méthyliques de colophane hydrogénée et désodorisée utilisés de préférence dans le cadre de l'invention ont comme caractéristiques techniques : une viscosité Brookfield à 25°C sans solvant de 6000 à 8000 m Pa.s ; une masse volumique à 20°C de 1,025g/cm3 ; un point d'inflammabilité supérieur à 170°C ; un indice de réfraction de 1,516 à 1,519 à 20°C ; un indice de saponification de 170 à 176 ; un indice d'acide de 9 mg KOH/g maximum. Etant hydrogénés, ces esters méthyliques de colophane hydrogénée et désodorisée ont une bonne résistance dans le temps ; un traitement de désodorisation leur assure une odeur très faible. On peut utiliser, par exemple, les produits HYDROGRAL M de D.R.T./GRANEL ou HERCOLYN D-E de HERCULES.

La colophane hydrogénée esterifiée à la glycérine et désodorisée utilisée de préférence dans le cadre de l'invention a comme caractéristiques techniques : un point de ramollissement, bille et anneau, de 76°C à 89°C ; un indice d'acide de 3 à 10 mg KOH/g ; une densité à 25°C de 1,06 Kg/dm3. On peut utiliser les références HYDROGRAL G-5 de D.R.T./GRANEL ou STAYBELITE ESTER 5-E de HERCULES.

Les mono-diglycérides d'acides gras qui font partie des compositions de la présente invention ont de préférence comme caractéristiques techniques : un indice d'iode de 0 à 2,0 g I2/100 g ; un indice d'acide de 0 à 2,0 mg KOH/g ; un contenu en alpha monoglycérides de 35 à 50 % ; un taux de glycérine libre de 0 à 2,0 % maximum. On peut utiliser un ou plusieurs produits/mono-diglycérides d'acides gras choisis parmi les références suivantes : EMULSIO-45 de S.I.O. ; PANALITE 40 HVK de A.D.M. ARKADY ; GRINDSTED MONO-DI HT 60 de DANISCO ; RADIAMULS MG 2142 ou RADIAMULS MG 2143 de FINA CHEMICALS.

Les monoglycérides acétylés avec un degré d'acétylation de 50 % utilisés dans les compositions qui font l'objet de la présente invention ont comme caractéristiques techniques : un degré d'acétylation de 48,5 à 51,5 % ; un indice d'iode de 3,0 g I2/100 g max ; un indice d'acide de 3,0 mg KOH/g max ; un indice de saponification de 278 à 291 ; un indice d'hydroxyle de 133 à 152 ; un point de fusion dans le domaine 41°C à 46°C. On peut utiliser, par exemple, les références RADIAMULS AMG 2050 de FINA CHEMICALS, GRINDSTED ACETEM 50-00 de DANISCO ou AME-50/03 de A.D.M. ARKADY.

Les monoglycérides acétylés avec un degré d'acétylation de 70 % utilisés dans les formulations qui font l'objet de cette invention ont comme caractéristiques techniques : un degré d'acétylation de 66,9 à 69,5 ; un indice d'iode de 3,0 g I2/100 g max ; un indice d'acide de 3,0 mg KOH/g max ; un indice de saponification de 317 à 330 ; un indice d'hydroxyle de 81 à 94 ; un point de fusion dans le domaine 37° à 40°C. On peut utiliser par exemple les produits suivants : AME-70/03 de A.D.M. ARKADY ; GRINDSTED ACETEM 70-00 de DANISCO ; RADIAMULS AMG 2021 de FINA CHEMICALS.

Les lécithines de soja qui peuvent être utilisées dans les compositions de la présente invention ont comme caractéristiques techniques : un indice d'acide de 20 à 30 mg KOH/g ; un taux d'insolubles dans l'acétone de 32 à 67 %. On peut utiliser, par exemple, les références suivantes : YELKIN 1018 de A.D.M. ou THERMOLEC 68 de A.D.M.

Le polyisobutylène qui peut être utilisé dans la présente invention a comme caractéristique technique propre un poids moléculaire de 10000 à 95000, de préférence entre 50000 et 80000. On peut utiliser, par exemple, la référence VISTANEX LM-MS de EXXON CHEMICALS.

Pour préparer une composition d'enrobage selon l'invention, on utilise de préférence un procédé dans lequel les monoglycérides acétylés à 50 % et à 70 % sont pré-mélangés à une température de 50°C - 60°C pour obtenir un pré-mélange liquide ; les mono-diglycérides d'acides gras sont introduits dans une enceinte munie de moyens d'agitation et maintenus à une température de 70°-80°C pendant 25-40 minutes sous agitation ; ledit prémélange liquide est introduit dans l'enceinte qui est ensuite maintenue sous agitation à une température d'environ 50°-60°C, la substance résineuse est incorporée progressivement au mélange constitué par ledit pré-mélange et les mono-diglycérides sous agitation jusqu'à obtention d'une composition homogène.

Pour préparer une composition d'enrobage selon l'invention incorporant du polyisobutylène, on utilise un procédé dans lequel le polyisobutylène est introduit dans une enceinte munie de moyens d'agitation et maintenue à une température supérieure d'environ 15°C au point de goutte Mettler du composant mono-diglycéride choisi ; les autres composants de la composition d'enrobage sont introduits ensuite, progressivement et sous agitation, dans ledit polyisobutylène de façon à obtenir une composition homogène.

A la suite des deux procédés décrits de façon générale ci-dessus, et de façon détaillée dans les trois exemples ci-dessous, la composition homogène est refroidie à température ambiante et mise sous une forme choisie parmi les plaques, les écailles et les pastilles.

### Exemple 1 :

200 g d'EMULSIO-45 (un mono-diglycéride d'acides gras) sont introduits dans un pétrin avec pales en Z. Le pétrin est chauffé à une température de 74°C à 76°C dans la double enveloppe. 200 g de RADIAMULS AMG 2021 (un monoglycéride acétylé à 70%) et 350 g de RADIAMULS AMG 2050 (un monoglycéride acétylé à 50%) sont associés préalablement dans un prémélange chauffé à une température de 55°C. Le prémélange des monoglycérides acétylés est ensuite transféré dans le pétrin ; le pétrin est mis en marche pendant 10 minutes. 100 g d'HYDROGRAL M (un ester méthylique de colophane hydrogénée et désodorisée) sont additionnés au pétrin par petites quantités avec le pétrin en marche. Finalement, 150 g d'oléine de palme hydrogénée sont ajoutés dans le pétrin. La préoccupation principale est de maintenir une incorporation progressive des autres composants dans le prémélange de mono-diglycérides d'une manière continue et homogène. Après un temps total de malaxage de 25 à 35 minutes, le pétrin est vidé de la composition et la composition est mise sous la forme de plaques, écailles ou pastilles.

### Exemple 2 :

230 g de VISTANEX LM-MS (polyisobutylène) sont chauffés à 70°C. Un pétrin à pales en Z est chauffé préalablement à une température de 74 à 76°C dans la double enveloppe. La totalité du VISTANEX LM-MS (230g) est introduite dans le pétrin à l'arrêt. Le pétrin est mis en marche pendant 5 minutes. Avec le pétrin en marche, 360 g d'EMULSIO-45 (un mono-diglycérides d'acides gras) sont introduits progressivement dans le pétrin. Une incorporation progressive du composant mono-diglycérides est nécessaire. Après l'incorporation totale de ce dérivé de matières grasses végétales, 205 g de matières grasses végétales hydrogénées et/ou interestérifiées sont introduits dans le pétrin en marche. L'incorporation de ces matières demande 10 minutes. Après cet intervalle de temps, on introduit dans la masse obtenue 205 g d'HERCOLYN D-E (un ester méthylique de colophane hydrogénée et désodorisée) et on continue le malaxage. Après un temps total de 25 minutes, la masse obtenue étant homogène, on arrête le malaxage et on vide la composition obtenue.

### Exemple 3 :

400 g de RADIAMULS MG 2143 (mono-diglycéride d'acides gras), sous forme d'écailles/flocons/poudre, solide à température ambiante, sont introduits dans un réacteur chimique chauffé à 72°C dans la double enveloppe. Le produit est maintenu sous agitation pendant une période de 25 à 40 minutes. Un mélange liquide à une température de 50°C à 58°C a préalablement été préparé contenant 300 g de GRINDSTED ACETEM 50-00 (monoglycérides acétylés à 50 %) et 100 g de GRINDSTED ACETEM 70-00 (monoglycérides acétylés à 70 %). Le mélange intermédiaire est maintenu à une température de 55°C pendant 15 minutes sous agitation. 200 g d'un mélange 50%/50% de HYDROGRAL M et de HERCOLYN D-E préalablement chauffé à 38°C est introduit dans le réacteur. La composition est maintenue en agitation à une température de 55°C à 58°C pendant 20 minutes. La composition finale est transférée dans une cuve de stockage ; ensuite cette composition finale objet de l'invention est mise sous la forme de plaques, écailles ou pastilles.

Les tableaux n°01 à 05 donnent quelques exemples de compositions selon l'invention.

Le tableau n°06 montre la teneur en solide ou "solid fat index" en pourcent des compositions A1 du tableau 01 et C2 du tableau 03, determinée par résonance magnétique nucléaire à basse résolution selon la méthode IUPAC 2.150.a

Le point de goutte Mettler de la composition A1 est de 47,8°C et celui de la composition C2 est de 47,6°C.

Les compositions d'enrobage, objet de la présente invention, présentent en général un point de goutte Mettler bas, de l'ordre de 48 à 52°C. Ces valeurs sont inférieures au point de goutte Mettler des formulations ternaires et quaternaires indiqués dans le tableau C du document EP 403 030.

Le fait que les compositions d'enrobage objet de la présente invention présentent en général un point de goutte Mettler bas permet leur utilisation à des températures du bain de trempage de l'ordre de 58° à 60°C seulement, voire moins, ce qui représente une économie d'énergie.

Les compositions selon la présente invention sont évaluées en vue de déterminer leur aptitude à enrober des fromages selon des tests, utilisés dans les essais pilotes des industries fromagères, très proches des procédés industriels d'enrobage. Le mode opératoire pour l'enrobage de fromage à pâte semi-dure de forme dite "ronde", avec un ruban d'ouverture est le suivant :
I - les fromages sont tempérés à 17-19°C pendant 4 heures.
II - on effectue leur trempage dans un premier bain d'une composition d'enrobage maintenue à une température de 53°-54°C. Le fromage est trempé pendant 1-2 secondes.
III - le fromage est retiré du bain et mis à égoutter pendant 4 à 5 secondes à température ambiante de 20° à 22°C. Pendant l'égouttage, la première couche d'enrobage se solidifie.
IV - on procède au trempage dans un deuxième bain pendant 1 à 2 secondes. La composition du deuxième bain peut être identique à celle du premier bain ou différente.
V - le fromage est retiré du bain et mis à égoutter pendant 4 à 5 secondes à température ambiante de 20° à 22°C. Pendant l'égouttage, l'enrobage se solidifie.
VI - on procède ensuite à une trempe froide par immersion dans de l'eau ayant une température de 6 à 8°C pendant 5 à 6 secondes.
VII - les fromages enrobés sont ensuite laissés à température ambiante pendant 10 minutes, ce qui correspond à la durée moyenne des opérations de conditionnement.
VIII - les fromages sont ensuite stockés au frais à une température entre +4 et +8°C.

Les propriétés physiques, mécaniques et visuelles des compositions d'enrobage sont évaluées :
1) sous forme de plaques, avant leur utilisation en tant que bain de trempage.
2) sous forme de fromage enrobé avant l'ouverture,
3) leur comportement en cours d'ouverture,
4) leur état après l'ouverture.

Les tableaux 7, 8 et 9 présentent les performances de plusieurs compositions suivant l'invention, les appréciations attribuées représentant la moyenne des valeurs d'une cinquantaine d'essais.

Les compositions d'enrobage selon la présente invention peuvent être utilisées indifféremment comme première couche de protection, suivie d'autres couches de même nature ou de nature différente ou comme couches extérieures.

Les essais exposés ci-dessous montrent que les compositions selon la présente invention peuvent être utilisés pour fabriquer un produit d'emballage en feuilles pouvant être substitué au produit bien connu sous le nom de "papier paraffiné".

### Exemple 4 : fabrication d'un papier imprégné sur une face

La composition utilisée est un mélange, à 0,7 part en poids de la composition A6 pour une part en poids de la composition A5 du tableau 1. La composition de ce mélange est donc :
- mono-diglycérides à 45% : 30%
- monoglycérides acétylés distillés à 70% : 19%
- monoglycérides acétylés distillés à 50% : 41%
- résine ester méthylique de colophane : 10 %

Cette composition est maintenue a une température de 65°-70°C dans un récipient plat, telle qu'une barquette métallique.

On fait glisser une feuille de papier sur la surface de la composition ; on laisse le papier et la composition en contact pendant 3-4 secondes. On retire le papier et on le maintient en position verticale pour permettre l'égouttage pendant 12 à 15 secondes.

### Exemple 5 : fabrication d'un papier imprégné double face

On utilise la même composition que dans l'exemple précédent. On plonge la feuille de papier dans la composition, en l'y maintenant pendant 3-4 secondes. On retire le papier et on le laisse s'égoutter pendant 20 à 25 secondes.

Les feuilles de papier imprégnées selon les procédures des exemples 4 et 5 sont testés selon les essais ci-dessous. Les tests sont effectués après une conservation de deux mois des papiers fabriqués selon les exemples 4 et 5.
1) Grammage : on détermine le poids du papier avant et après imprégnation, en grammes par mètre carré.
2) Test d'enroulage : on enroule une feuille de papier imprégné de dimensions 8 x 15 cm sur un cylindre ayant un diamètre de 7 mm. On considère que l'échantillon est parfait quand il s'enroule facilement, sans modification de son aspect extérieur, c'est-à-dire sans écaillage et sans trace de pliage;
3) Test de pliage : on plie et on déplie la feuille de papier imprégné une dizaine de fois. On considère qu'un échantillon est "très bon" s'il résiste à ce test, avec un écaillage minime.
4) Test d'écaillage : on soumet la feuille de papier imprégnée à un froissement manuel intensif et on évalue l'aspect visuel de la feuille de papier après dépliage.
5) Test de résistance au froid : on conserve la feuille de papier imprégné au réfrigérateur à environ + 5°C pendant 72 heures. A la sortie du réfrigérateur, on effectue le test n°2 d'enroulage sur un cylindre de 7 mm.

Les résultats des tests sont donnés dans le tableau 10.

Les avantages et propriétés remarquables des compositions selon l'invention sont illustrées par les résultats d'essais exposés ci-dessus et résumés dans les tableaux n°07 à 10.

Ils montrent que les compositions selon l'invention peuvent être utilisées pour enrober un produit alimentaire en appliquant directement la composition sur ledit produit alimentaire et que ces compositions peuvent également servir à l'emballage d'un produit alimentaire en appliquant la composition sur un support auxiliaire, telle qu'une feuille de papier, et en se servant du produit en feuille ainsi fabriqué pour emballer le produit alimentaire.

L'opération d'enrobage peut être réalisée non seulement par trempage, mais aussi par aspersion ou par badigeonnage.

Les compositions d'enrobage selon la présente invention sont compatibles avec d'autres matières pour la protection et/ou l'emballage de produits alimentaires, pharmaceutiques ou cosmétiques, ce qui permet une utilisation en association avec d'autres matières de protection ou d'emballage d'origines diverses.

Elles sont exemptes d'hydrocarbures minéraux et des dérivés d'hydrocarbures minéraux, en accord avec les tendances réglementaires et administratives actuelles.

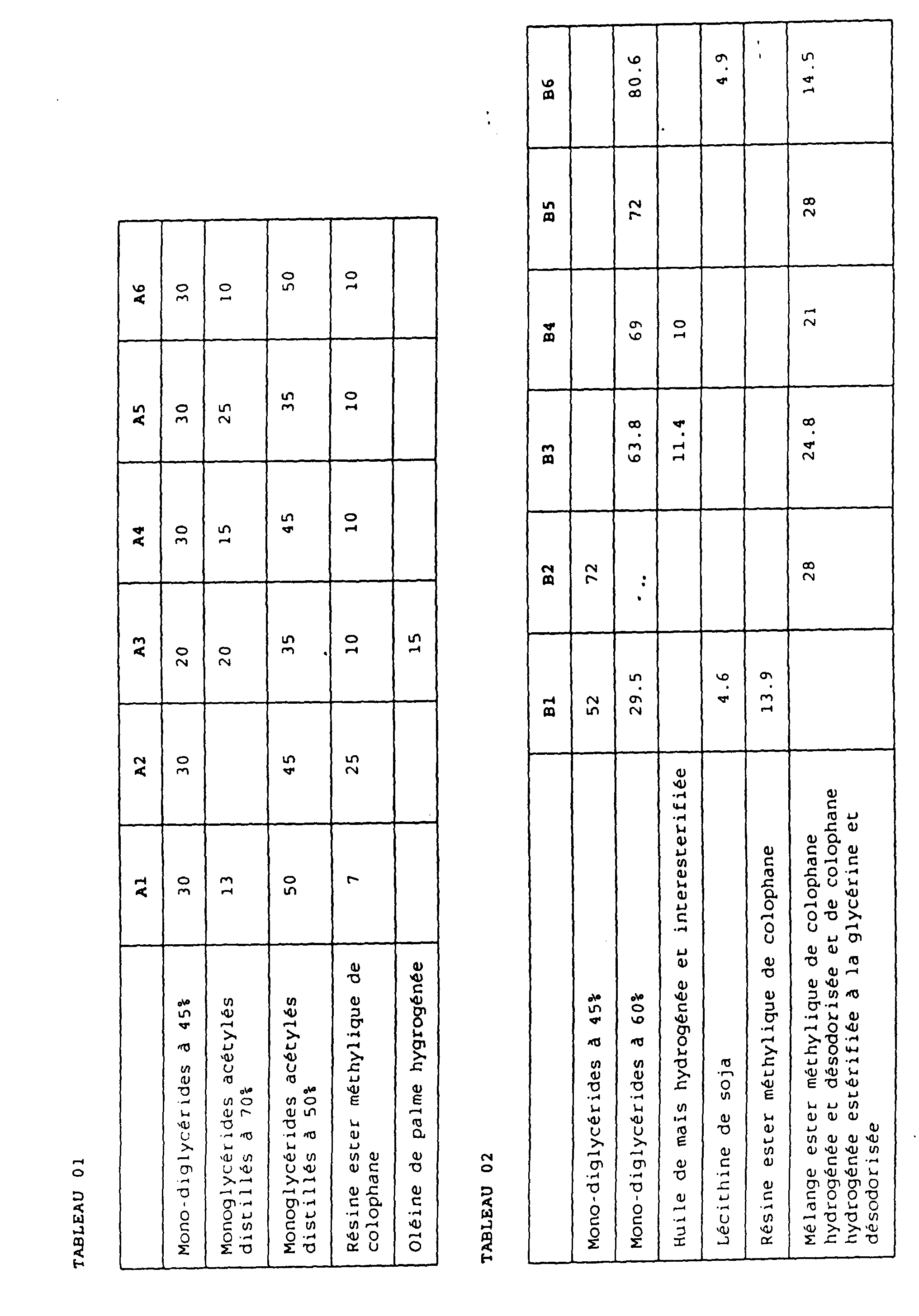

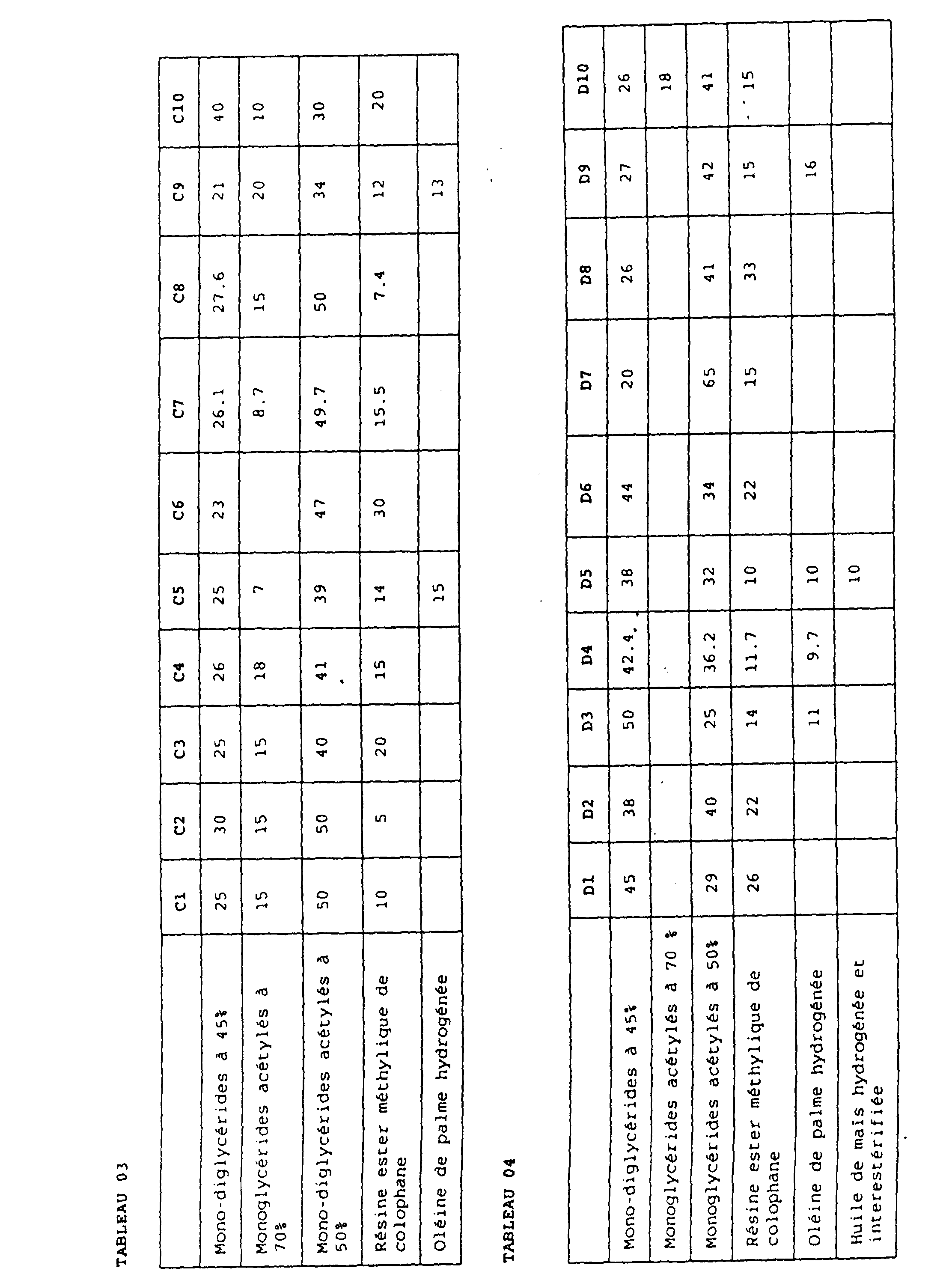

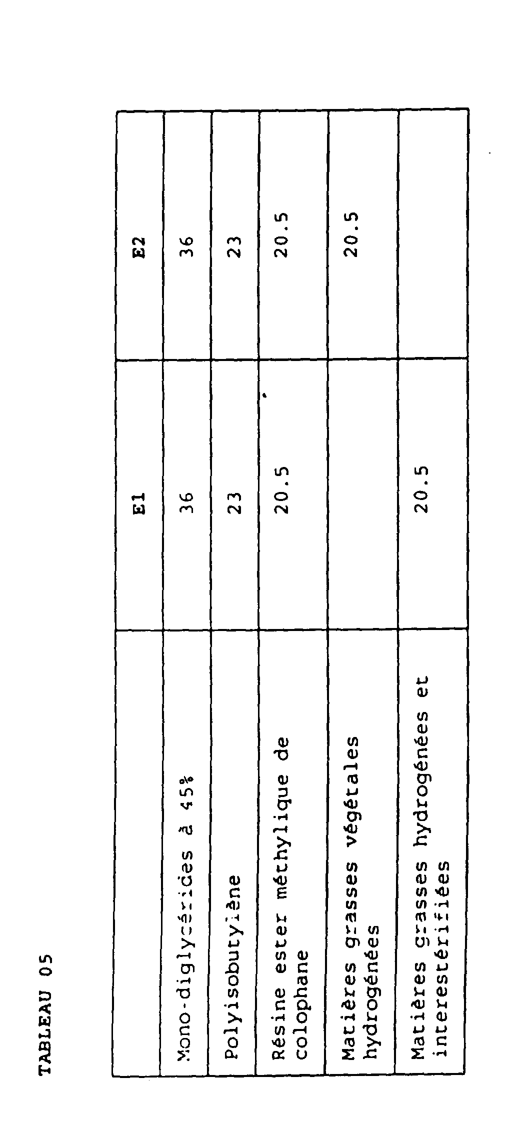

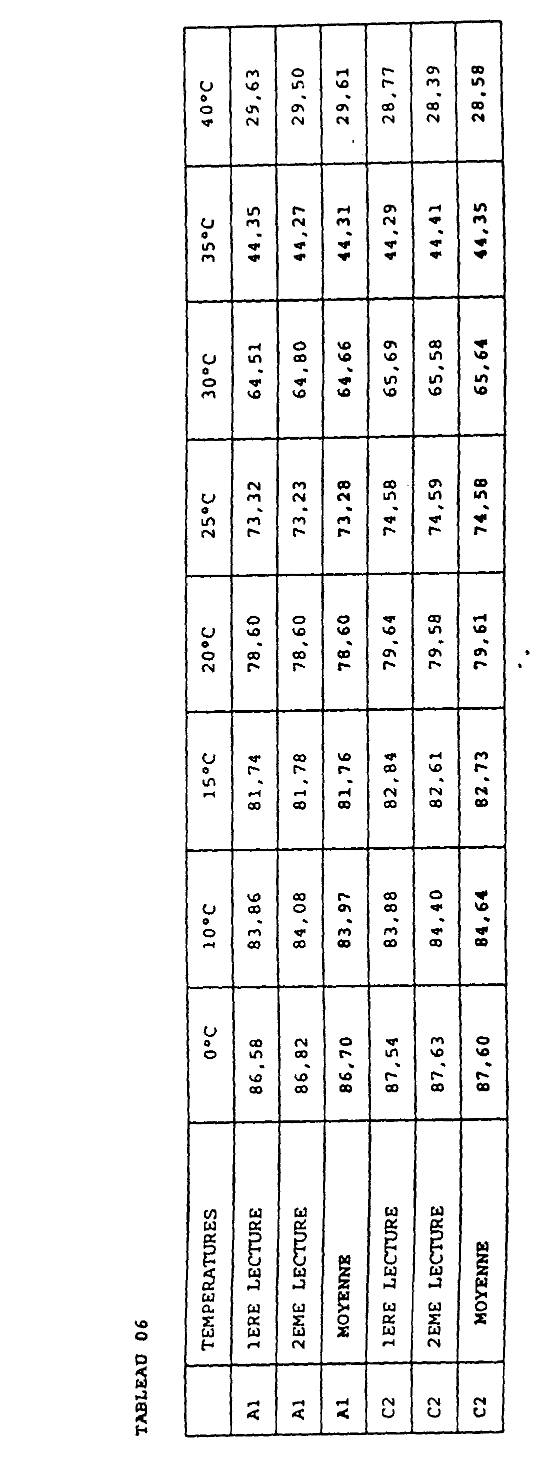

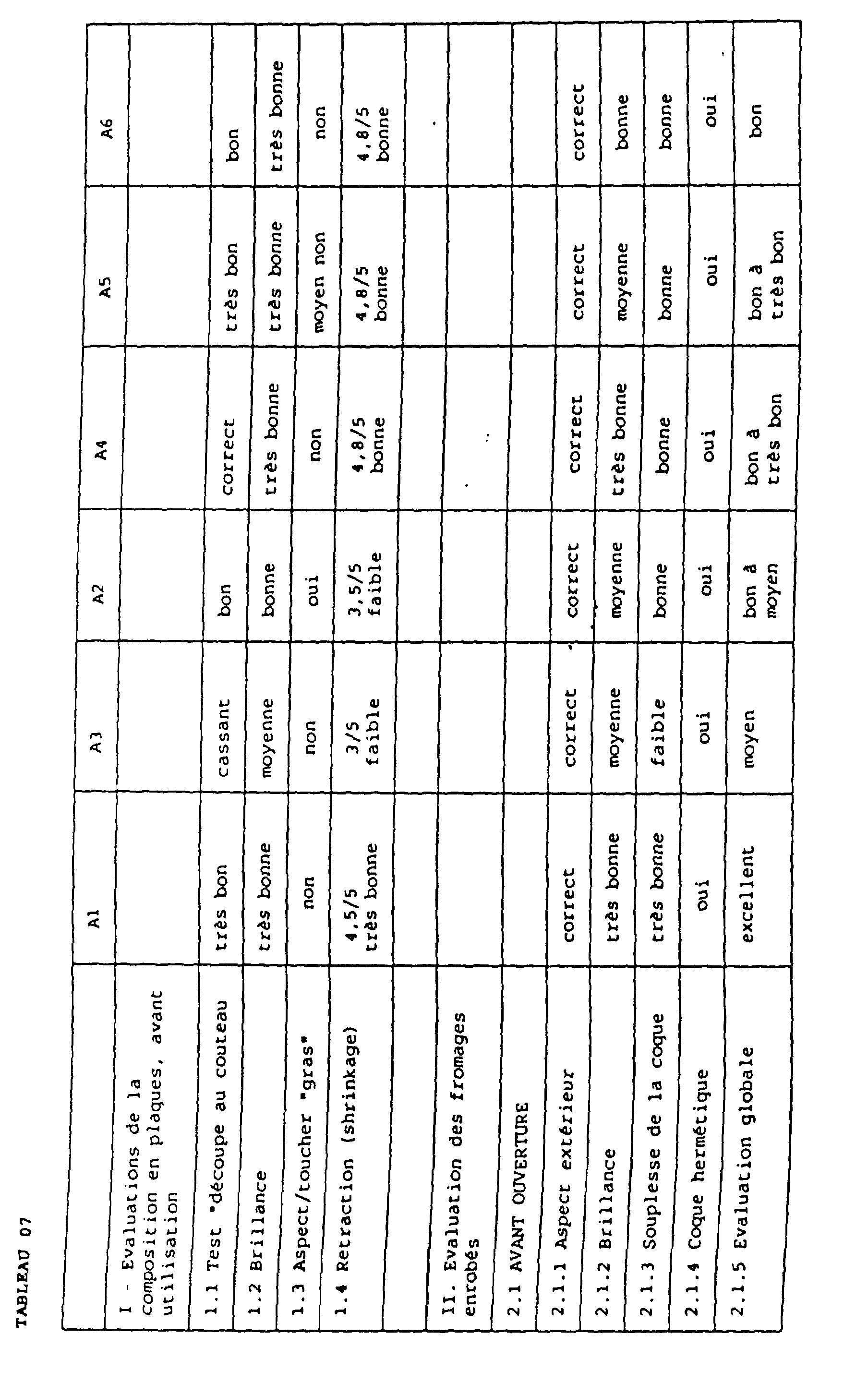

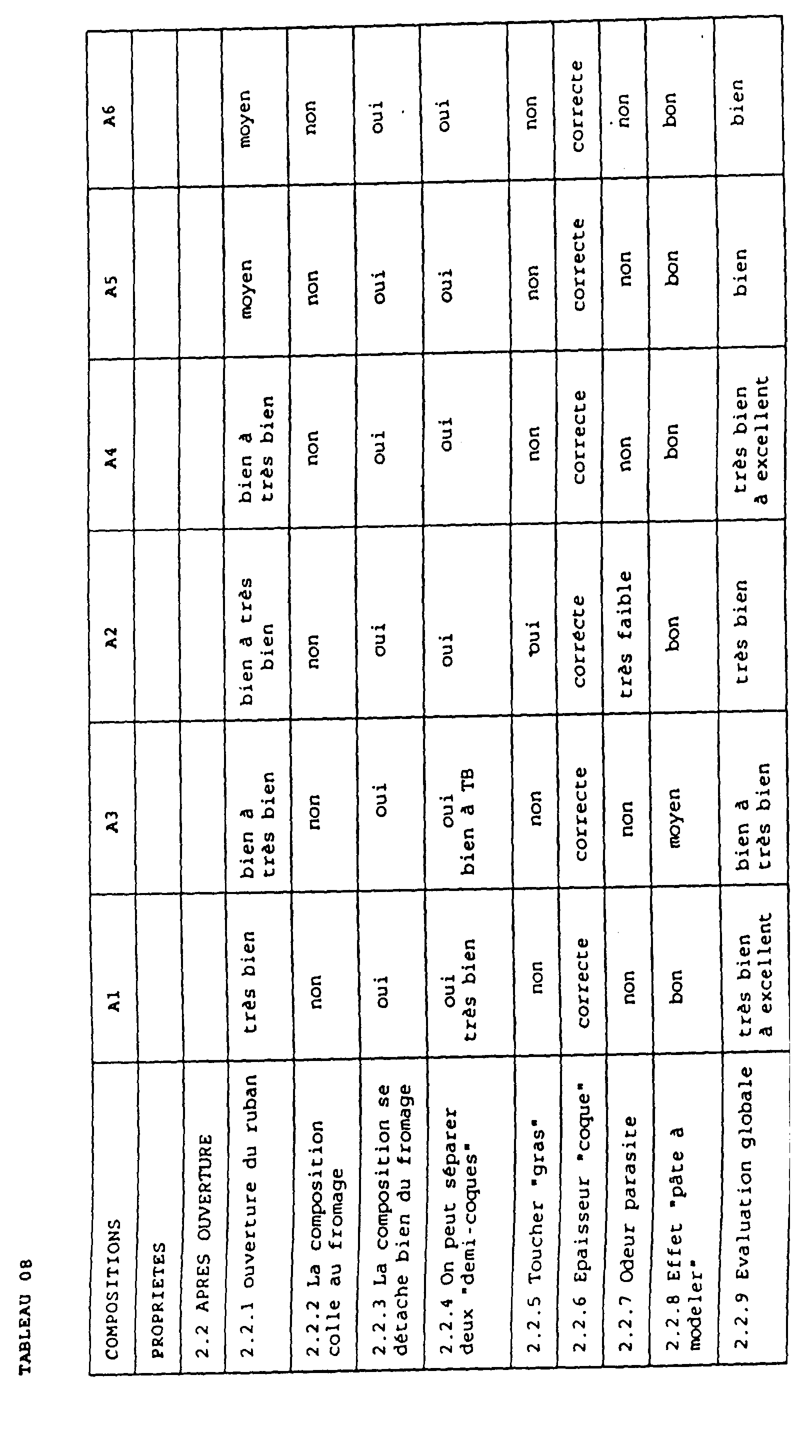

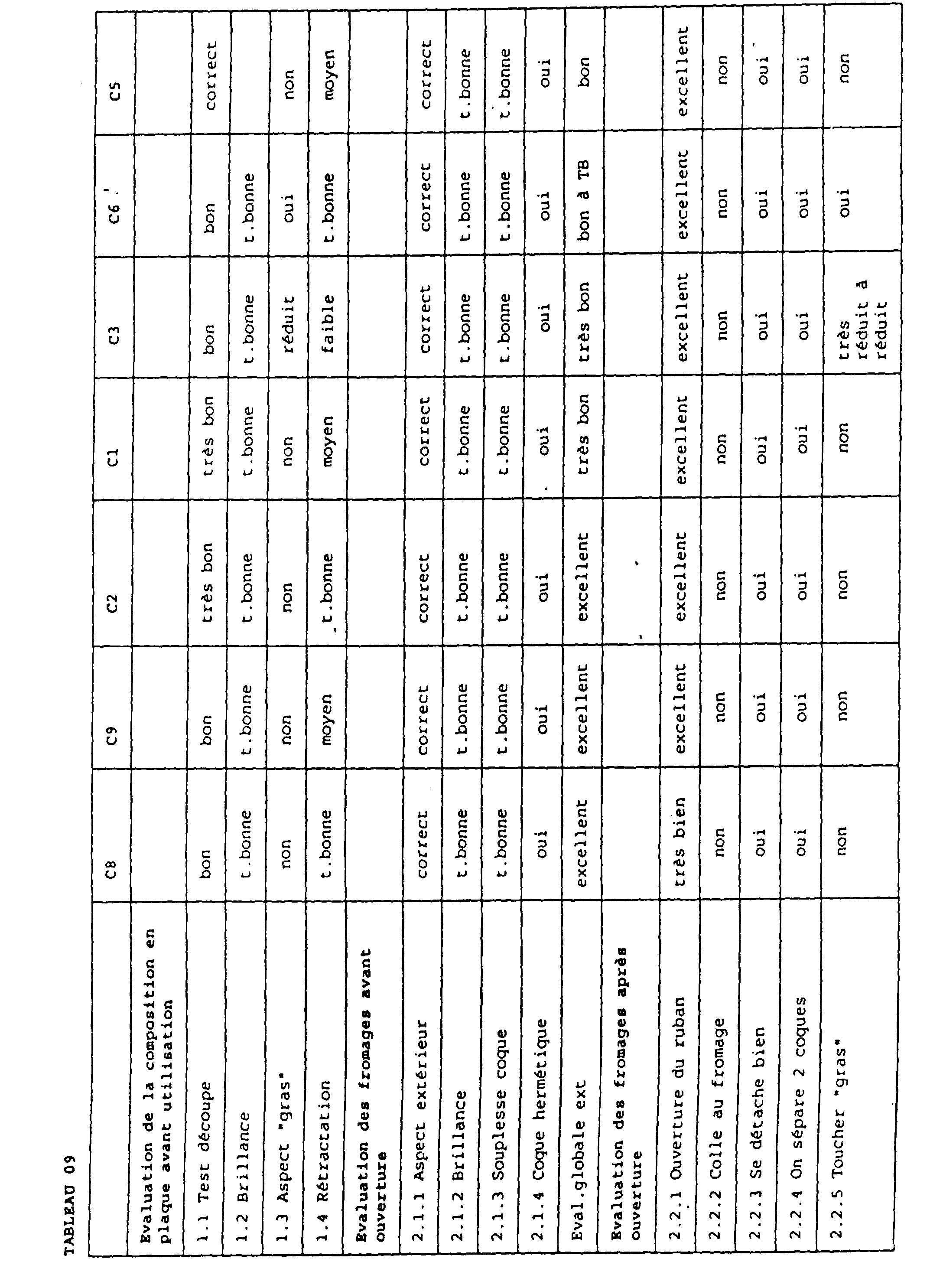

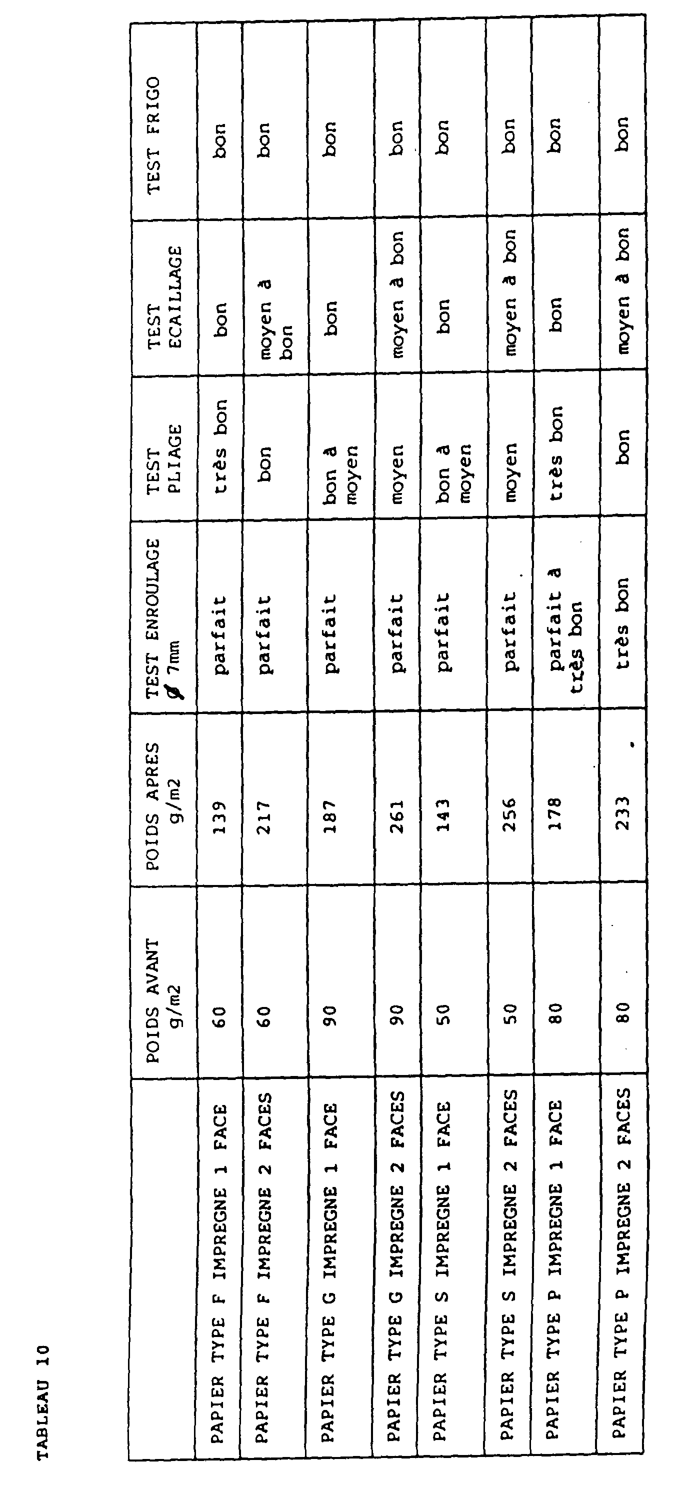

## Revendications

1. Composition d'enrobage caractérisée en ce qu'elle comprend
de 20 à 80 % en poids de mono-diglycérides d'acides gras et
de 3 % à 30 % en poids d'une substance résineuse choisie parmi les esters méthyliques de colophane hydrogénée et désodorisée, la colophane hydrogénée estérifiée au glycérol et désodorisée, et leurs mélanges.

2. Composition d'enrobage selon la revendication 1 caractérisé en ce que lesdits mono-diglycérides d'acides gras sont choisis parmi les produits fabriqués à partir de matières grasses végétales ou animales.

3. Composition d'enrobale caractérisée en ce que lesdits mono-diglycérides d'acides gras sont choisis parmi les produits fabriqués à partir d'acides gras.

4. Composition d'enrobage selon l'une des revendications 1 à 3, caractérisée en ce que lesdits acides gras sont choisis parmi l'acide myristique (C14:0), le palmitique (C16:0), le stéarique (C18:0), l'arachidique (C20:0) et le béhénique (C22:0) .

5. Composition d'enrobage selon l'une des revendications 1 à 4 caractérisée en ce qu'elle comprend entre 20 % à 50 % en poids desdits mono-diglycérides, et de 25 % à 70 % en poids d'un ou plusieurs glycérides acétylés choisis parmi les monoglycérides acétylés à 50 % et les monoglycérides acétylés à 70 %.

6. Composition d'enrobage selon la revendication 5, caractérisée en ce qu'elle contient une association de monoglycérides acétylés à 50 % et de monoglycérides acétylés à 70 % et en ce que le rapport pondéral monoglycérides acétylés à 50 %/monoglycérides acétylés à 70 % est compris entre 0,6 et 5,7 et de préférence entre 1,4 et 3,8.

7. Composition d'enrobage selon l'une des revendications précédentes caractérisée en ce qu'elle contient de plus une ou plusieurs substances choisies parmi la lécithine de soja, l'oléine de palme hydrogénée, l'huile de maïs hydrogénée, l'huile de palmiste hydrogénée, les matières grasses végétales hydrogénées et interesterifiées.

8. Composition d'enrobage selon l'une quelconque des revendications précédentes, caractérisée en ce qu'elle contient de 10 % à 36 % en poids de polyisobutylène de poids moléculaire compris entre 10 000 et 95 000 et de préférence entre 50 000 et 80 000.

9. Composition d'enrobage selon l'une des revendications 1-7 caractérisée en ce qu'elle comprend 25 % de monoglycérides d'acides gras, 7 % de monoglycérides acétylés à 70 %, 39 % de monoglycérides acétylés à 50 %, 14 % d'un ester méthylique de colophane hydrogénée et désodorisée et 15 % d'oléine de palme hydrogénée.

10. Composition d'enrobage selon l'une des revendications 1-7 caractérisée en ce qu'elle comprend de 28 à 30 % de mono-diglycérides d'acides gras, de 13 à 15 % de monoglycérides acétylés à 70 %, de 48 à 50 % de monoglycérides acétylés à 50 % et de 5 à 7 % d'un ester méthylique de colophane hydrogénée et désodorisée.

11. Composition d'enrobage selon l'une des revendications 1-7 caractérisée en ce qu'elle comprend 30 % de mono-diglycérides d'acides gras, 10 % de monoglycérides acétylés à 70 %, 50 % de monoglycérides acétylés à 50 % et 10 % d'un ester méthylique de colophane hydrogénée et désodorisée.

12. Composition d'enrobage selon l'une des revendications 1-7 caractérisée en ce qu'elle comprend 27.6 % de mono-diglycérides d'acides gras, 8.7 % de monoglycérides acétylés à 70 %, 50 % de monoglycérides acétylés à 50 % et 7.4 % d'un ester méthylique de colophane hydrogénée et désodorisée.

13. Procédé de préparation d'une composition d'enrobage selon l'une des revendications 6 ou 7, caractérisée en ce que les monoglycérides acétylés à 50 % et à 70 % sont pré-mélangés à une température de 50°C - 60°C pour obtenir un pré-mélange liquide, que les mono-diglycérides d'acides gras sont introduits dans une enceinte munie de moyens d'agitation et maintenus à une température de 70°-80°C pendant 25-40 minutes sous agitation, que ledit prémélange liquide est introduit dans l'enceinte qui est ensuite maintenue sous agitation à une température de 50°-60°C, que ladite substance résineuse est incorporée progressivement au mélange constitué par ledit pré-mélange et les mono-diglycérides sous agitation jusqu'à obtention d'une composition homogène.

14. Procédé de préparation d'une composition d'enrobage selon la revendication 8, caractérisé en ce que le polyisobutylène est introduit dans une enceinte munie de moyens d'agitation et maintenue à une température supérieure d'environ 15°C au point de goutte Mettler du composant mono-diglycéride choisi ; que les autres composants de la composition d'enrobage sont introduits ensuite, progressivement et sous agitation, dans ledit polyisobutylène de façon à obtenir une composition homogène.

15. Procédé selon la revendication 13 ou la revendication 14, caractérisé en ce que ladite composition homogène est refroidie à température ambiante et mise sous une forme choisie parmi les plaques, les écailles et les pastilles.

16. Procédé selon l'une des revendications 13 à 15, caractérisé en ce que ladite enceinte munie de moyens d'agitation est un pétrin à pales en "Z".

17. Utilisation d'une composition d'enrobage selon l'une des revendications 1 à 12 pour l'enrobage d'un produit alimentaire.

18. Utilisation selon la revendication 17, caractérisée en ce que ledit produit alimentaire est un fromage ou une préparation fromagère.

19. Utilisation selon la revendication 17 ou 18, caractérisée en ce qu'une couche d'enrobage est obtenue par trempage dudit produit alimentaire dans ladite composition à l'état fondu.

20. Utilisation selon la revendication 17 ou 18, caractérisée en ce qu'une couche d'enrobage est obtenue par aspersion dudit produit alimentaire à l'aide de ladite composition d'enrobage à l'état fondu.

21. Utilisation d'une composition d'enrobage selon l'une des revendications 1 à 12 pour la fabrication d'un produit d'emballage en feuilles.

22. Utilisation selon la revendication 21 caractérisée en ce qu'un matériau de support en feuille, choisi parmi les papiers, les cartons , les textiles tissés et non tissés est imprégné au moyen de ladite composition d'enrobage.

23. Utilisation selon la revendication 22, caractérisée en ce que l'imprégnation est effectuée ladite composition d'enrobage étant à l'état fondu.

24. Utilisation d'une composition d'enrobage selon l'une des revendications 1 à 12 pour la protection d'un produit cosmétique.

25. Utilisation d'une composition d'enrobage selon l'une des revendications 1 à 12 pour la protection d'un produit pharmaceutique.
